## Europäisches Patentamt

## European Patent Office

## Office européen des brevets

(11) Veröffentlichungsnummer: **0 192 080**
**A2**

(12) **EUROPÄISCHE PATENTANMELDUNG**

(21) Anmeldenummer: **86100942.1**

(22) Anmeldetag: **24.01.86**

(51) Int. Cl.⁴: **A 61 K 9/20**

(30) Priorität: **22.02.85 DE 3506276**

(43) Veröffentlichungstag der Anmeldung:
**27.08.86 Patentblatt 86/35**

(84) Benannte Vertragsstaaten:
**AT BE CH DE FR GB IT LI NL SE**

(71) Anmelder: **Meggle Milchindustrie GmbH & Co. KG**
**Megglestrasse 6 - 12**
**D-8094 Reitmehring(DE)**

(72) Erfinder: **Bauer, Kurt H., Prof. Dr.**
**Im Finkeler 4**
**D-7800 Freiburg(DE)**

(72) Erfinder: **Pritzwald-Stegmann, Bernd, Dipl.-Ing.**
**Föhrenstrasse 4**
**D-8094 Reitmehring(DE)**

(72) Erfinder: **Luft, Werner**
**Föhrenstrasse 5**
**D-8094 Reitmehring(DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel Möhlstrasse 22**
**D-8000 München 80(DE)**

(54) **Direkttablettiermittel.**

(57) Es wird ein Direkttablettiermittel sowie ein Verfahren zu seiner Herstellung beschrieben, das im wesentlichen aus Lactose und Cellulose mit einem Verhältnis von Lactose zu Cellulose, bezogen auf das Gewicht von 95 bis 40 : 5 bis 60 besteht, wobei die Lactose teils in kristalliner, teils in amorpher Form und die Cellulose als feinteiliges Pulver vorliegt.

Croydon Printing Company Ltd.

- 1 -

# B e s c h r e i b u n g

Die Erfindung betrifft ein Direkttablettiermittel und Verfahren zu seiner Herstellung.

Es sind verschiedene Verfahren zur Tablettenherstellung bekannt. Besonders wünschenswert ist die Direkttablettierung. Dazu wird der pharmazeutische Wirkstoff mit dem Trägermaterial vermischt und direkt tablettiert. Dieses Verfahren ist besonders einfach, da es keine Zwischenstufen zur Herstellung eines Kerns oder Granulats erfordert. Für die Direkttablettierung sind jedoch viele der bekannten Trägermaterialien nicht geeignet. Das Trägermaterial muß sowohl eine gute Fließfähigkeit, als auch eine gute Komprimierbarkeit bei geringem Druck besitzen. Die Preßlinge müssen eine hohe Härte und Abriebfestigkeit besitzen und gleichzeitig aber auch bei Kontakt mit dem Magensaft schnell zerfallen. Diese Anforderungen werden zur Zeit weitgehend von mikrokristalliner Cellulose, die als Hilfsstoff zur Direkttablettierung verwendet wird, erfüllt. Nachteil dabei ist es, daß mikrokristalline Cellulose schwierig herzustellen ist und daher auch teuer und nicht sehr wirtschaftlich ist.

Aufgabe der Erfindung war es daher, ein Mittel für die Direkttablettierung zu schaffen, das einerseits die Anforderungen, die an ein derartiges Material gestellt werden, erfüllt, das aber andererseits kostengünstiger als die teure mikrokristalline Cellulose ist.

Gelöst wird diese Aufgabe durch ein Direkttablettiermittel, bestehend im wesentlichen aus Lactose und Cellulose mit einem Verhältnis von Lactose zu Cellulose, bezogen auf das Gewicht von 95 bis 40:5 bis 60, wobei die Lactose teils in kristalliner, teils in amorpher
Form und Cellulose als feinteiliges Pulver vorliegt.

Das erfindungsgemäße Mittel eignet sich sehr gut für
die Direkttablettierung. Es hat ausgezeichnete Fließeigenschaften. Die daraus hergestellten Preßlinge sind
sehr hart und zerfallen schnell.

Das erfindungsgemäße Direkttablettiermittel besteht im
wesentlichen aus Lactose und Cellulose. Dabei liegt das
Verhältnis von Lactose zu Cellulose, bezogen auf das
Gewicht, im Bereich von 95 bis 40 Teilen Lactose zu 5
bis 60 Teilen Cellulose. Enthält das Mittel mehr als
95 % Lactose, so sind die daraus geformten Tabletten
nicht hart genug. Die Verwendung von mehr als 60 %
Cellulose ist unwirtschaftlich, da eine weitere Verbesserung der Eigenschaften nicht mehr erreicht wird.
Bevorzugt beträgt das Verhältnis von Lactose zu Cellulose 80 bis 65 Teile Lactose zu 20 bis 35 Teile Cellulose. Besonders bevorzugt ist eine Mischung aus 75
Teilen Lactose und 25 Teilen Cellulose.

Es ist wesentlich, daß in dem erfindungsgemäßen Direkttablettiermittel die Lactose teils in kristalliner,
teils in amorpher Form vorliegt. Die Kombination von
Lactose in kristalliner und amorpher Form und von feinteiliger Cellulose bedingt die guten Eigenschaften des
erfindungsgemäßen Mittels.

Dem erfindungsgemäßen Direkttablettiermittel können
noch übliche Zusatzstoffe zugesetzt werden. Als übliche
Zusatzstoffe werden bei der Tablettenherstellung
Sprengmittel, Aromatisierungsmittel, Stabilisatoren,
Füllmittel, Farbstoffe und/oder Gleitmittel verwendet.

Gemäß einer ersten Methode zur Herstellung des erfindungsgemäßen Direkttablettiermittels wird Cellulosepulver
mit einer heißen Lösung von z. B. 50 bis 60 % Lactose
in Wasser vermischt, die Mischung abgekühlt und die
erhaltene Masse granuliert und anschließend getrocknet.

Die heiße Lactoselösung hat vorzugsweise eine Temperatur
zwischen 60 und 100°C. Bevorzugt liegt die Temperatur
der Lactoselösung im Bereich von 90 bis 95°C.

Die Trocknung der granulierten Masse erfolgt mit üblichen
Trocknungsverfahren. Bevorzugt ist die Trocknung im
Fließbett.

Als Cellulose wird sehr feines Cellulosepulver nach der
Pharmacopoe verwendet. Bevorzugt ist die Cellulose mit
einer Teilchengröße von <100 µm. Die Verwendung von
mikrokristalliner Cellulose ist nicht erforderlich, sie
ist jedoch möglich.

Gemäß einer weiteren Methode ist es möglich, das erfindungsgemäße Direkttablettiermittel herzustellen, indem
man eine mikrokristalline Lactose mit Cellulosepulver
im Verhältnis von 95 bis 40 Gewichtsteilen Lactose zu 5
bis 60 Gewichtsteilen Cellulosepulver vermischt, die
Mischung in kaltem Wasser aufschlämmt und anschließend
sprühtrocknet.

Als mikrokristalline Lactose wird hierbei eine Lactose
verstanden mit einer Teilchengröße von weniger als
100 µm. Bevorzugt wird eine Lactose verwendet, deren
Kristalle überwiegend eine Teilchengröße von weniger
als 50 µm besitzen. Besonders bevorzugt wird eine
Lactose verwendet, deren Kristalle überwiegend eine
Teilchengröße von weniger als 32 µm haben.

Als Cellulose wird eine sehr feine pulverförmige Cellulose nach Pharmacopoe verwendet. Bevorzugt ist die Cellulose mit einer Teilchengröße von < 100 µm. Die Verwendung von mikrokristalliner Cellulose ist nicht erforderlich, sie ist jedoch möglich.

Mit dem erfindungsgemäßen Direkttablettiermittel können Tabletten hergestellt werden, die eine große Härte besitzen und schnell zerfallen. Überraschenderweise haben die aus dem erfindungsgemäßen Direkttablettiermittel hergestellten Tabletten bei einem gegebenen Preßdruck eine größere Härte als Preßlinge, die aus feiner pulverförmiger Cellulose oder aus mikrokristalliner Cellulose hergestellt werden.

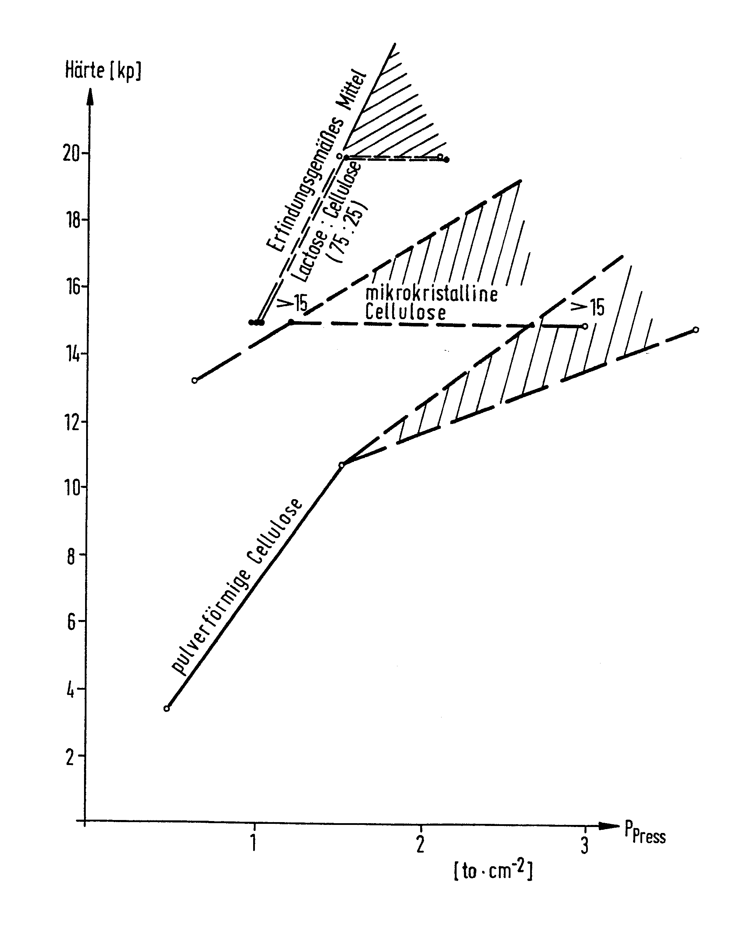

Fig. 1 zeigt eine graphische Darstellung, in der die Härte von Preßlingen aus feiner pulverförmiger Cellulose, mikrokristalliner Cellulose und dem erfindungsgemäßen Direkttablettiermittel gegen den Preßdruck aufgetragen sind. Dieser Figur ist eindeutig zu entnehmen, daß mit dem erfindungsgemäßen Tablettiermittel insbesondere bei dem erwünschten mittleren Preßdruck besonders harte Preßlinge erhalten werden.

B e i s p i e l     1

880 kg kaltes Wasser, 358,3 kg feinkristalline Lactose, bei der 95 % eine Teilchengröße von weniger als 32 µm besitzen, und 125 kg pulverförmige Cellulose werden in einem geeigneten Behälter so gemischt, daß dem vorgelegten Wasser erst die Lactose und danach die Cellulose zugegeben und das ganze einige Minuten gut vermischt wird. Anschließend wird die erhaltene Aufschlämmung in einem Trockenturm versprüht, dem Luft mit einer Temperatur von ungefähr 150°C zugeführt wird. Das getrocknete Produkt kann direkt verwendet werden.

B e i s p i e l    2

Cellulosepulver wurde mit einer heißen Lösung von
50%iger Lactose in Wasser vermischt, die Mischung wurde
abkühlen gelassen, die erhaltene Masse granuliert und
anschließend getrocknet. In der Masse waren 75% Lactose
und 25 % Cellulose enthalten. Die Siebanalyse des
Produktes war:

Teilchen          $>$500 µm    24,2 %,
           400 bis 500 µm    14,6 %,
           315 bis 400 µm    12,0 %,
           200 bis 315 µm    13,8 %,
           100 bis 200 µm    23,2 %,
                $<$100 µm    12,0 %.

Es wurden Tabletten gepreßt mit einem Preßdruck von
I     1,0 t/cm²,
II    1,5 t/cm²,
III   2,2 t/cm².

Die erhaltenen Tabletten hatten eine Härte von
I:     5,0 kp,
II:    9,2 kp,
III: 14,4 kp.

Die Zerfallsgeschwindigkeit der Tabletten betrug bei
37°C in Wasser
I     34 sec.,
II    3 min, 35 sec.,
III   18 Minuten.

Beispiel  3

Aus einem durch Sprühtrocknung hergestellten Direkttablettiermittel aus 75% Lactose und 25% Cellulose
wurden Tabletten hergestellt. Der Preßdruck betrug:

I   1,0 t/cm²
II   1,5 t/cm²
III  2,1 t/cm².

Die Tablettenhärte der Preßlinge betrug

I   15,0 kp,
II   mehr als 20,0 kp,
III  mehr als 20,0 kp.

Die Zerfallsgeschwindigkeit der Tabletten war bei
37°C in Wasser:

I    etwa 15 Minuten,
II und III etwa eine Stunde.

Beispiel  4

Es wurden Tabletten gepreßt aus mikrokristalliner
Cellulose, aus feinem Cellulosepulver, aus Lactose und
aus einem Direkttablettiermittel gemäß der Erfindung
mit 75 % Lactose und 25% Cellulose. Die Ergebnisse sind
in der folgenden Tabelle aufgeführt:

## T a b e l l e  1

| | V e r g l e i c h | | | Erfindung |
| --- | --- | --- | --- | --- |
| | mikrokrist. Cellulose | pulverf. Cellulose | Lactose D 80 | 75 % Lactose 25 % Cellulose |
| **Siebanalyse (g)** | | | | |
| $>$800 (µm) | | 0,1 | | |
| 630–800 | | 0,3 | | |
| 500–630 | | 0,3 | 0,2 | |
| 400–500 | | 0,3 | 1,0 | |
| 315–400 | 0,1 | 0,7 | 26,0 | |
| 200–315 | 4,7 | 2,9 | 35,5 | 16,2 |
| 100–200 | 28,7 | 33,9 | 23,7 | 30,4 |
| $<$100 | 66,6 | 61,6 | 13,8 | 53,4 |
| **Preßdruck (t/cm²)** | | | | |
| I | 0,63 | 0,48 | 0,25 | 1,1 |
| II | 1,20 | 1,48 | 1,50 | 1,6 |
| III | 2,98 | 4,08 | 4,40 | 2,2 |
| **Tablettenhärte (kp)** | | | | |
| I | 13,3 | 3,5 | 0,5 | 8,4 |
| II | 15,0 | 10,8 | 1,5 | 15,4 |
| III | 15,0 | 15,0 | 5,0 | 18,8 |
| **Tablettenzerfall ($H_2O$/37°C)** | | | | |
| I | 2 min 23 sec | 15 min | 23 sec | 19 sec |
| II | 14 min 30 sec | 15 min | 25 sec | 2 min 4 sec |
| III | 15 min | 15 min | 60 sec | 13 min 30 sec |

Der Vergleich zeigt, daß mit dem erfindungsgemäßen
Tablettiermittel Tabletten erhalten werden, deren Härte
mit Tabletten aus mikrokristalliner Cellulose vergleichbar ist und deren Auflösegeschwindigkeit sehr gut ist.

## Patentansprüche

1. Direkttablettiermittel, bestehend im wesentlichen aus Lactose und Cellulose mit einem Verhältnis von Lactose zu Cellulose, bezogen auf das Gewicht von 95 bis 40 : 5 bis 60, wobei die Lactose teils in kristalliner, teils in amorpher Form und die Cellulose als feinteiliges Pulver vorliegt.

2. Direkttablettiermittel nach Anspruch 1, d a d u r c h   g e k e n n z e i c h n e t , daß das Verhältnis von Lactose zu Cellulose, bezogen auf das Gewicht, im Bereich von 65 bis 80 : 35 bis 20 liegt.

3. Direkttablettiermittel nach einem der Ansprüche 1 oder 2, d a d u r c h   g e k e n n z e i c h n e t ,   daß es zusätzlich als Zusatzstoffe noch Sprengmittel, Aromatisierungsmittel, Stabilisatoren, Füllmittel, Farbstoffe und/oder Gleitmittel enthält.

4. Verfahren zur Herstellung eines Direkttablettiermittels nach Anspruch 1, d a d u r c h   g e k e n n z e i c h n e t ,   daß man Cellulosepulver mit einer heißen Lösung von 50 bis 60 % Lactose in Wasser vermischt, die Mischung abkühlen läßt, die erhaltene Masse granuliert und anschließend trocknet.

5. Verfahren zur Herstellung eines Direkttablettiermittels nach Anspruch 1,          d a d u r c h
g e k e n n z e i c h n e t ,       daß man eine
Mischung aus mikrokristalliner Lactose und
Cellulosepulver im Verhältnis von 95 bis 40 : 5
bis 60, bezogen auf das Gewicht, in kaltem Wasser
aufschlämmt und anschließend sprühtrocknet.

6. Verfahren nach Anspruch 5,          d a d u r c h
g e k e n n z e i c h n e t ,        daß man als
mikrokristalline Lactose eine Lactose mit einer
Teilchengröße von weniger als 100 µm verwendet.

7. Verfahren nach Anspruch 5 oder 6,
d a d u r c h          g e k e n n z e i c h n e t ,
daß eine Lactose verwendet wird, die überwiegend
eine Teilchengröße von weniger als 50 µm hat.

8. Verfahren nach einem der Ansprüche 4 bis 7,
d a d u r c h          g e k e n n z e i c h n e t ,
daß man eine Lactose verwendet, bei der der überwiegende Teil der Teilchen eine Größe von unter
32 µm hat.

9. Verfahren nach einem der Ansprüche 4 bis 8,
d a d u r c h          g e k e n n z e i c h n e t ,
daß man der Mischung aus Cellulose und Lactose
noch als übliche Zusatzstoffe Sprengmittel, Aromatisierungsmittel, Stabilisatoren, Füllmittel,
Farbstoffe und/oder Gleitmittel zusetzt.